# EUROPÄISCHE PATENTSCHRIFT

(11) **EP 3 041 517 B1**
(45) Veröffentlichungstag und Bekanntmachung des Hinweises auf die Patenterteilung: **05.07.2017**
(21) Anmeldenummer: 14758957.6
(22) Anmeldetag: 04.09.2014
(51) Int. Cl.: A61L 2/00, A61B 17/3203, B24C 5/02

(54) **HANDSTÜCK ZUR BEHANDLUNG VON WUNDEN**
HANDPIECE FOR TREATING WOUNDS
PIÈCE À MAIN DESTINÉE AU TRAITEMENT DE BLESSURES

(30) Priorität: 06.09.2013 EP 13183380
(43) Veröffentlichungstag der Anmeldung: 13.07.2016
(73) Patentinhaber: MEDAXIS AG, 6340 Baar (CH)
(72) Erfinder: BERNHARD, Jérôme, CH-8003 Zürich (CH); CHRISTEN, Lukas, CH-6004 Luzern (CH); DE PABLO PEÑA, Albora, CH-8037 Zürich (CH); LIMACHER, Kuno, CH-6312 Steinhausen (CH); SCHUG, Martin, CH-Meggen 6045 (CH); SIMON, Maryline, CH-6314 Unterägeri (CH); WIDMER, Beat, CH-6005 Luzern (CH); WIGET, Roland, CH-6403 Küssnacht am Rigi (CH)
(74) Vertreter: Clerc, Natalia
(86) Internationale Anmeldenummer: PCT/EP2014/068848
(87) Internationale Veröffentlichungsnummer: WO 2015/032863

(56) Entgegenhaltungen:
- EP-A1- 2 251 142
- WO-A1-2006/097133
- WO-A2-2006/107343
- WO-A2-2010/123627
- US-A1- 2013 270 445

## Beschreibung

### TECHNISCHES GEBIET

Die vorliegende Erfindung betrifft ein Handstück sowie ein Verfahren zur Behandlung, insbesondere zur Reinigung von Wunden mit einem Fluidstrahl.

### STAND DER TECHNIK

Als Wundreinigung / Debridement und Wundspülung / Wundtoilette bezeichnet man medizinische Vorgehen zur Entfernung von infiziertem, geschädigtem oder abgestorbenem (nekrotischem) Gewebe aus Geschwüren, Verbrennungs- und anderen Wunden oder bei Organzerfall. Es existieren zahlreiche generelle Ansätze für die Wundreinigung, z.B. mechanisch-scharfe unter der Verwendung eines Skalpells oder eines scharfen Löffels, enzymatische oder chemische, autolytische, biochirurgische, mechanische unter der Verwendung von Pads und mit Fluidstrahl.

Die EP 2 251 142 zeigt ein Handstück zur Reinigung von Wunden mit einem Hochdruck-Mikro-Fluidstrahl, wobei eine Austrittsöffnung am vorderen Ende des Handstücks angeordnet ist, durch welche ein Fluidstrahl austreten kann.

WO 97/02058 offenbart ein Handstück zur Wundreinigung mittels eines Wasserstrahls, wobei das zur Reinigung verwendete Wasser vorgängig mittels UV-Bestrahlung sterilisiert wird.

WO 2010/123627 offenbart eine kombinierte Vorrichtung mit einer UV-A oder UV-C Lichtquelle, einem optischen Erkennungseinheit und einer Flüssigkeitszufuhr- und Absaugeinheit. Alle drei Einheiten sind an ein gemeinsames Kabel angeschlossen, welche an seinem freien Ende drei voneinander getrennte Ausgänge zur Bilderkennung, UV-Lichtbestrahlung und Fluidzufuhr und -Absaugung aufweist. Die UV-Lichtbestrahlung wird dabei zu Therapiezwecken verwendet.

Bei der Wundreinigung mittels eines Fluidstrahls werden Beläge oder Partikel aus der Wunde entfernt, wobei Aerosole entstehen. Es ist wichtig sicherzustellen, dass die Umgebung nicht durch diese Aerosole kontaminiert wird, da dies eine Gefährdung für den Patienten oder das Operationspersonal darstellt. Aus dem Stand der Technik sind zahlreiche Verfahren bekannt, die die Kontaminierung der Umgebung durch die Aerosole reduzieren oder verhindern. Solche Verfahren, wie z.B. Flüssigkeitsrückführung, abschirmendes Behandlungszelt mit Abzugsschleuse oder die Anordnung des Fluidstrahls und der Absaugung in einer Abdeckhaube sind in der WO 2008/074284 erwähnt.

Je nach der Art, Grösse und Lage der zu reinigenden Wunde ist der Einsatz eines Systems mit einer Abdeckhaube nur beschränkt möglich. Ist die Haube zu klein, können entweder nur kleine Wunden damit behandelt werden oder die Haube muss auf einem Teil der Wunde aufgesetzt werden, was für den Patienten schmerzhaft und für die Wundheilung unvorteilhaft sein kann. Wenn jedoch die Haube zu gross ist, dann gestaltet sich die Abdichtung gegenüber dem umliegenden Gewebe schwierig. Wird ein Behandlungszelt verwendet, so vereinfacht dies zwar das Abdichten, jedoch sind solche Zelte unhandlich, da sie eine geringe Eigenstabilität aufweisen und die Sicht auf die zu reinigende Wunde verringert.

### DARSTELLUNG DER ERFINDUNG

Es ist daher eine Aufgabe der vorliegenden Erfindung, die Gefahr einer Kontaminierung der Umgebung während der Behandlung, insbesondere der Reinigung einer Wunde zu verringern.

Diese Aufgabe wird erfindungsgemäss durch ein System mit den Merkmalen des Patentanspruchs 1 gelöst.

Die Lichtquelle ist ein lichtemittierendes Element. Das Licht wird in einer Ausführungsform in der Lichtquelle selber erzeugt. In einer anderen Ausführungsform ist die Lichtquelle eine Austrittsöffnung eines Lichtleiters. Oberflächenflächen, welche genügend glänzend ausgebildet sind, um einfallendes Licht zu reflektieren, jedoch nicht Teil eines Lichtleiters sind, werden im Sinne dieses Textes und auch der Fachwelt nicht als Lichtquellen verstanden.

Die mindestens eine Lichtquelle kann separat zum Handstück angeordnet sein. Vorzugsweise ist sie jedoch im oder am Handstück angeordnet, so dass das Handstück selber das System bildet.

Der Fluidstrahl kann aus einer oder aus mehreren Austrittsöffnungen austreten.

Vorzugsweise ist der Fluidstrahl ein Mikro-Fluidstrahl, insbesondere ein Hochdruck-Mikro-Fluidstrahl. Als Hockdruck wird hier insbesondere der Druckbereich von 100 bis 300 bar verstanden. Es ist jedoch auch denkbar, dass der Fluidstrahl mit einem Druck von weniger als 100 bar erzeugt wird. Das Fluid ist üblicherweise eine wässrige Lösung bzw. eine Behandlungslösung, wie z.B. eine sterile Kochsalzlösung. Vorzugsweise weist der Fluidstrahl einen Durchmesser von circa 0,05 mm bis 0,15 mm bei Austritt aus der Austrittsöffnung auf.

Das UV-Licht lässt sich während der Wundreinigung durch das Fluid zur Minimierung von lebensfähigen Keimen im Behandlungsraum und vorzugsweise auch in der Wunde einsetzen. Aerosole, welche während der Wundreinigung entstehen, lassen sich gleich vor Ort und während der Reinigung der Wunde bestrahlen und dekontaminieren und dies, bevor sie die Umgebung kontaminieren können. Die Wirkung ist erhöht, wenn das Licht den Fluidstrahl insbesondere im Bereich seines Auftritts auf die Wunde umgibt. Vorzugsweise umgibt das Licht den Fluidstrahl mindestens in diesem Bereich vollständig.

Durch die Bestrahlung der Wunde während der Reinigung kann die Wundheilung gefördert werden. Zudem kann die UV Bestrahlung eine Alternative zu antimikrobiellen Salben und Antibiotika darstellen.

Ein weiterer Vorteil ist, dass insbesondere bei kleineren Wunden keine Zelte, Hauben oder Absaugungen mehr notwendig sind, so dass die Sicht auf die Wunde unbeschränkt möglich ist. Des Weiteren werden durch die Vereinfachung der verwendeten Mittel die Behandlungszeit und die Behandlungskosten reduziert.

Das UV-Licht (Ultraviolett-Licht) weist vorzugsweise einen UV-C Lichtanteil (100 - 280 nm) auf. In einer bevorzugten Ausführungsform ist lediglich oder mindestens zu einem überwiegenden Teil UV-C Licht vorhanden.

Zusätzlich oder alternativ weist das UV-Licht einen UV-A oder einen UV-B Lichtanteil auf oder besteht ausschliesslich aus UV-A oder UV-B.

Vorzugsweise handelt es sich beim UV-Licht um kohärentes Licht, vorzugsweise um Laserlicht.

Vorzugsweise ist die Lichtquelle eine UV-C Lichtquelle im Bereich 200 bis 280 nm. In diesem Bereich weist das Licht eine erhöhte Entkeimungswirkung auf. Ein besonders wirkungsvoller Bereich der Wellenlänge liegt bei ca. 260 Nanometer. Vorzugsweise werden daher Lichtquellen mit einer Wellenlänge von 254 Nanometer verwendet.

Vorzugsweise ist das Licht der Lichtquelle im Handstück erzeugbar. Dies hat den Vorteil, dass die Intensität der Strahlung nicht durch z.B. Lichtleiter abgeschwächt wird. Es ist jedoch auch denkbar, das Licht in einem hinteren Bereich des Handstücks zu erzeugen und kurze Lichtleiter einzusetzen, um das Licht im Wesentlichen in Strahlrichtung zu leiten.

Die Lichtquelle ist vorzugsweise in einem fluidstrahlaustrittsnahen Bereich des vorderen Endes angeordnet. Für Lichtquellen gleicher Stärke gilt: je näher die Lichtquelle am zu bestrahlenden Bereich liegt, desto höher ist die Energiedichte im bestrahlten Bereich und desto wirkungsvoller ist die Bestrahlung.

Die Lichtleistung der Lichtquelle kann fix oder regelbar sein. Die elektrische Speisung der Lichtquelle kann durch eine Lichtquellenleitung bzw. Elektroleitung erfolgen, welche eine externe Stromquelle mit dem Handstück verbindet oder kann durch im Handstück vorgesehene elektrische Speicher, z.B. Batterien oder Akkumulatoren, erfolgen.

Vorzugsweise verläuft der Fluidstrahl benachbart zur Austrittsöffnung strahlabwärts zumindest teilweise innerhalb des ausgesandten Lichts. Vorzugsweise verläuft er bis zu seinem Auftreffen auf die Wunde im Licht. Je näher die Lichtquelle zur Wunde hingeführt wird, desto wirkungsvoller ist die Bestrahlung der Aerosole.

Vorzugsweise ist die mindestens eine Lichtquelle die Austrittöffnung umgebend am oder im vorderen Ende des Handstücks angeordnet. Sie kann jedoch auch in einem Adapter oder an einem Halter angeordnet sein, welcher am Handstück anordnungsbar ist. Diese Anordnung am Handstück kann lösbar oder fest sein.

Die mindestens eine Lichtquelle weist vorzugsweise einen Durchmesser auf, der um ein Vielfaches grösser als der Querschnitt des Fluidstrahls ist. Je näher die mindestens eine Lichtquelle am Fluidstrahl angeordnet ist und je grösser die Lichtquelle im Vergleich zum Durchmesser des Fluidstrahls ist, desto kleiner ist der durch den Fluidstrahl verursachte Schatten. Hinsichtlich der Bestrahlung der durch die Wundreinigung entstehenden Aerosole bzw. der Wundoberfläche ist ein kleiner Schattenbereich vorteilhaft.

Durch die Anzahl, die Anordnung, die Ausgestaltung der Lichtquellen sowie die Wahl einer allfälliger Strahlrichtungs- und/oder Aufweitungsoptik kann eine optimale Bestrahlung der Aerosole bzw. der Wundoberfläche erreicht werden.

Das Handstück kann sich im vorderen Ende verjüngen, um die Bestrahlung möglichst nicht zu behindern oder es kann sich weiten, um grössere Lichtquellen aufnehmen zu können. Das Handstück weist vorzugsweise eine vordere Stirnfläche auf, in welcher die Austrittsöffnung des Fluidstrahls angeordnet ist, wobei die mindestens eine Lichtquelle bezüglich der vorderen Stirnfläche, in Austrittsrichtung des Fluidstrahls, zu dieser zurückversetzt, mit dieser fluchtend oder zu dieser vorstehend angeordnet ist. Je nach Anwendungsfall ist es vorteilhaft, dass die Lichtquellen möglichst klein sind, d.h. dass sie möglichst die Sicht des Benutzers nicht behindern, oder dass die Lichtquellen möglichst gross sind, d.h. dass sie eine hohe Strahlungsintensität erzeugen. Die Art und Grösse der Lichtquellen bedingt dementsprechende Aufnahmen im Handstück oder im Adapterteil. Je nach benötigter Grösse der Lichtquelle und der benötigten Sicht auf die zu reinigende Wunde ist es vorteilhaft, die Lichtquellen bezüglich der vorderen Stirnfläche anders anzuordnen.

Vorzugsweise weist das vordere Ende des Handstücks eine Ausnehmung zur Aufnahme der Lichtquelle auf. Die Lichtquellen sind somit gut im Handstück integriert und zumindest teilweise vor äusseren Einwirkungen geschützt.

Die Ausnehmung weitet sich vorzugsweise in Austrittsrichtung des Fluidstrahls. Durch die Weitung der Ausnehmung wird das Licht nicht durch das Handstück behindert und ein Lichtkegel ist ausbildbar, der einen Bereich bestrahlt, der ein Vielfaches der Fläche des Querschnitts des Fluidstrahls darstellt. Vorzugsweise weitet sich die Ausnehmung in allen Richtungen, damit ein möglichst grosser Lichtkegel ausbildbar ist.

Wenn sich das vordere Ende des Handstücks in Austrittsrichtung des Fluidstrahls weitet, lassen sich grössere Lichtquellen einsetzen. Die Weitung kann gleichmässig am Umfang sein. Sie kann jedoch auch nur in den Bereichen mit den Lichtquellen realisiert sein. So lässt sich ein Kompromiss zwischen einer einfachen Bauweise und einer guten Sichtbarkeit der Wunde durch den Bediener erreichen.

Es können eine, zwei, drei, vier, fünf, sechs oder mehr Lichtquellen vorhanden sein. Durch die Anzahl, die Anordnung und die Regelung der Leistung der Lichtquellen lässt sich die Strahlungsintensität beeinflussen.

Vorzugsweise weist die mindestens eine Lichtquelle einen Lichtaustrittsbereich auf, welcher rund, oval, quadratisch, rechteckig, vieleckig oder torusförmig ausgebildet ist. Durch den Einsatz von handelsüblichen Lichtquellen können die Kosten für Spezialanfertigungen eingespart werden.

Vorzugsweise ist die Austrittsöffnung zentrisch im vorderen Ende angeordnet. Durch die zentrische Anordnung lässt sich das Handstück im Wesentlichen rotationssymmetrisch ausbilden, was für den Bediener eine vereinfachte Handhabung zur Folge hat, da es keine Rolle spielt, wie das Handstück gehalten wird.

Die Lichtquelle ist vorzugsweise eine Leuchtdiode (LED), eine auf einer Leiterplatte (PCB) angeordnete LED oder eine in einer integrierten Leiterplatte (ICB) integrierte LED. Leuchtdioden zeichnen sich durch eine hohe Lebensdauer und eine Unempfindlichkeit gegenüber Erschütterungen aus. Sie setzen die eingesetzte Energie effektiver als andere Lichtquellen in Licht um und produzieren dabei weniger Wärme und sie benötigen nur eine geringe Betriebsspannung.

In einer Ausführungsform der Erfindung weist das vordere Ende einen Lichtleiter auf, welcher von der Lichtquelle emittiertes Licht, im Wesentlichen in Richtung des Fluidstrahls verteilt. Durch den Einsatz eines Lichtleiters kann das durch die Lichtquelle erzeugte Licht spezifisch gerichtet werden. Die Form der Lichtquelle hat dann einen geringeren Einfluss auf die Ausbildung des jeweiligen Lichtkegels. Ebenfalls können bestimmte Bereiche intensiver bestrahlt werden als andere. Es ist so auch möglich Bereiche zu bestrahlen, die auf direktem Wege nicht bestrahlbar sind.

Vorzugsweise weist das vordere Ende eine Düse auf, welche die Austrittsöffnung bildet bzw. mit dieser fluchtet. Vorzugsweise ist die Lichtquelle benachbart zur Düse angeordnet. Die Düse bestimmt die Qualität sowie die Form des Fluidstrahls Die Anordnung der Lichtquellen benachbart zur Düse optimiert dank der Nähe zum Fluidstrahl die Bestrahlbarkeit der Aerosole durch die Lichtquellen.

Vorzugsweise ist das Handstück steif ausgebildet.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Darstellung einer ersten Ausführungsform eines erfindungsgemässen Systems zur Reinigung von Wunden mit Fluidstrahl, mit Lichtkegel und Fluidstrahl;
- Fig. 2: eine Darstellung einer durch ein Handstück gemäss Figur 1 beleuchteten Fläche mit Fluidstrahl;
- Fig. 3: eine zentrale Schnittansicht eines vorderen Endes eines Handstücks gemäss Figur 1 mit Lichtkegel und Fluidstrahl;
- Fig. 4: eine vergrösserte perspektivische Darstellung eines vorderen Endes eines Handstücks gemäss Figur 1 mit Lichtkegel und Fluidstrahl;
- Fig. 5: eine perspektivische Darstellung einer zweiten Ausführungsform eines erfindungsgemässen Systems zur Reinigung von Wunden mit Fluidstrahl, mit Lichtkegel und Fluidstrahl;
- Fig. 6: eine Darstellung einer durch ein Handstück gemäss Figur 5 beleuchteten Fläche mit Fluidstrahl;
- Fig. 7: eine zentrale Schnittansicht eines vorderen Endes eines Handstücks gemäss Figur 5 mit Lichtkegel und Fluidstrahl;
- Fig. 8: eine vergrösserte perspektivische Darstellung eines vorderen Endes eines Handstücks gemäss Figur 5 mit Lichtkegel und Fluidstrahl;
- Fig. 9: eine perspektivische Darstellung einer dritten Ausführungsform eines erfindungsgemässen Systems zur Reinigung von Wunden mit Fluidstrahl, mit Lichtkegel und Fluidstrahl;
- Fig. 10: eine Darstellung einer durch ein Handstück gemäss Figur 9 beleuchteten Fläche mit Fluidstrahl;
- Fig. 11: eine zentrale Schnittansicht eines vorderen Endes eines Handstücks gemäss Figur 9 mit Lichtkegel und Fluidstrahl;
- Fig. 12: eine vergrösserte perspektivische Darstellung eines vorderen Endes eines Handstücks gemäss Figur 9 mit Lichtkegel und Fluidstrahl;
- Fig. 13: eine perspektivische Darstellung einer vierten Ausführungsform eines erfindungsgemässen Systems zur Reinigung von Wunden mit Fluidstrahl, mit Lichtkegel und Fluidstrahl;
- Fig. 14: eine Darstellung einer durch ein Handstück gemäss Figur 13 beleuchteten Fläche mit Fluidstrahl;
- Fig. 15: eine zentrale Schnittansicht eines vorderen Endes eines Handstücks gemäss Figur 13 mit Lichtkegel und Fluidstrahl;
- Fig. 16: eine vergrösserte perspektivische Darstellung eines vorderen Endes eines Handstücks gemäss Figur 13 mit Lichtkegel und Fluidstrahl;
- Fig. 17: eine perspektivische Darstellung einer fünften Ausführungsform eines erfindungsgemässen Systems zur Reinigung von Wunden mit Fluidstrahl, mit Lichtkegel und Fluidstrahl; sowie
- Fig. 18: eine zentrale Schnittansicht eines vorderen Endes eines Handstücks gemäss Figur 17 mit Lichtkegel und Fluidstrahl.

### BESCHREIBUNG BEVORZUGTER AUSFÜHRUNGSFORMEN

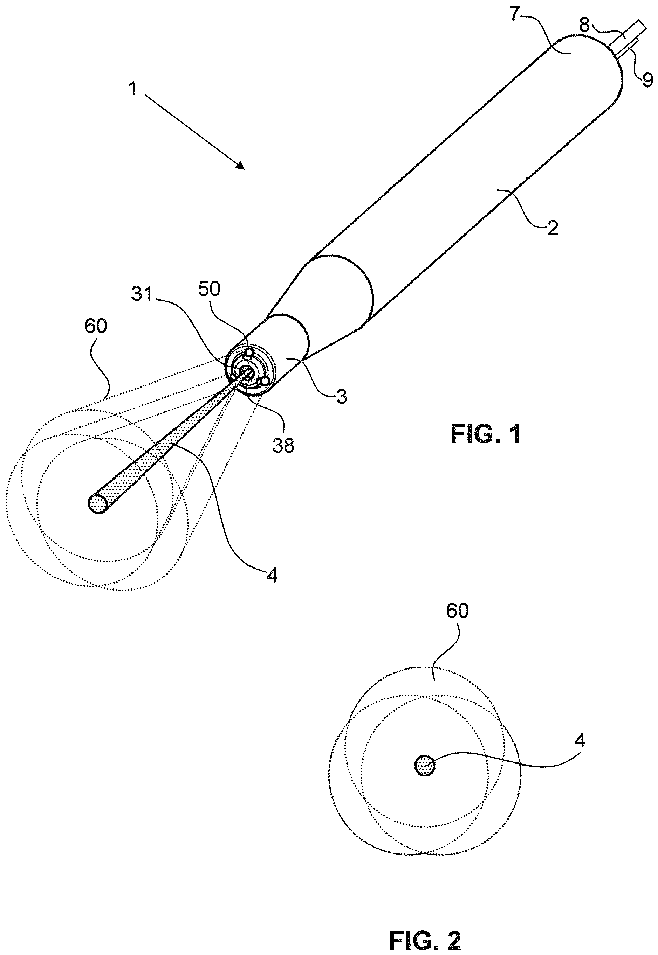

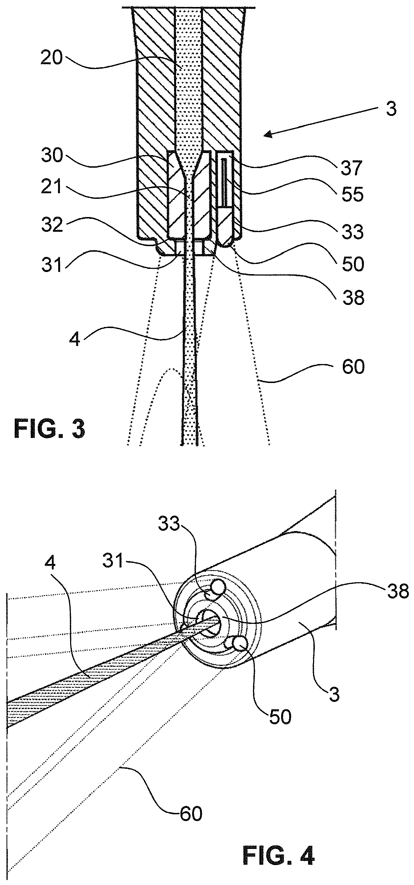

Die Figur 1 zeigt eine perspektivische Darstellung einer ersten Ausführungsform eines erfindungsgemässen Systems zur Behandlung, insbesondere zur Reinigung von Wunden mit Fluidstrahl, mit ersten Lichtkegeln 60 und einen Fluidstrahl 4, wobei das System in dieser Ausführungsform ein Handstück 1 ist. Figur 4 zeigt eine perspektivische Darstellung eines vorderen Endes 3 des Handstücks 1. Das Handstück 1 weist einen im Wesentlichen zylindrischen Grundkörper 2 mit dem vorderen Ende 3 und einem hinteren Ende 7 auf. Das vordere Ende 3 weist eine Austrittsöffnung 31 zum Austritt des Fluidstrahls 4 auf. Die Austrittsöffnung 31 ist zentral in einer vorderen Stirnfläche 38 des Handstücks 1 angeordnet. Das Handstück 1 ist derart ausgebildet, dass der Fluidstrahl 4 im Wesentlichen kollinear zur Mittelachse des Grundkörpers 2 aus der Austrittsöffnung 31 austritt. Am vorderen Ende 3 sind vorzugsweise drei erste Lichtquellen 50 angeordnet. Die ersten Lichtquellen 50 sind gleichmässig über den Umfang des vorderen Endes 3 um die Austrittsöffnung 31 verteilt. Die ersten Lichtquellen 50 sind derart ausgebildet, dass ein Bereich, in Strahlrichtung vor dem Handstück 1, bestrahlbar ist. Vorzugsweise ist der im Einsatz umliegende und diesem benachbarte Bereich des Fluidstrahls 4 vollständig bestrahlbar, so dass möglichst alle entstehenden Aerosole vom Lichtstrahl erfasst werden.

Die Figur 2 zeigt eine Darstellung einer durch das Handstück 1 gemäss Figur 1 beleuchteten Fläche mit dem Fluidstrahl 4. Dies entspricht einem Strahlungsquerschnitt durch den Fluidstrahl 4 und die ersten Lichtkegel 60, strahlabwärts der Austrittsöffnung 31, während des Einsatzes. Obwohl sich die Verhältnisse mit dem Abstand zur Austrittsöffnung 31 ändern, bleibt der Fluidstrahl 4 im Wesentlichen in einem Zentrum der sich überlappenden Bereiche der ersten Lichtkegel 60 angeordnet. Erste Ausnehmungen 33 zur Aufnahme der ersten Lichtquellen 50 bilden Lichtaustrittsbereiche, welche in dieser Ausführungsform im Wesentlichen rund ausgebildet sind. Die ersten Lichtkegel 60 bestrahlen daher jeweils eine im Wesentlichen kreisförmige Fläche. Zusammen mit Figur 1 ist erkennbar, dass die ersten Lichtkegel 60 den Fluidstrahl 4 vollständig umhüllen.

Die Figur 1 zeigt weiter, dass das hintere Ende 7 eine Fluidleitung 8 und eine Elektroleitung 9 aufweist, wobei die Fluidleitung 8 die Versorgung des Handstücks 1 mit Fluid, vorzugsweise eine wässrige Lösung bzw. eine Behandlungslösung, wie z.B. eine sterile Kochsalzlösung, sicherstellt und die Elektroleitung 9 zumindest die Stromzufuhr der Lichtquellen 50 sicherstellt. Die nachfolgenden Ausführungsformen des erfindungsgemässen Handstücks 1 weisen ebenfalls eine Druckleitung 8 und eine Elektroleitung 9 am hinteren Ende 7 auf, welche jedoch in diesen Figuren nicht dargestellt sind.

Die Figur 3 zeigt eine zentrale Schnittansicht des vorderen Endes 3 des Handstücks 1 gemäss Figur 1 mit Lichtkegel 60 und Fluidstrahl 4. Das vordere Ende 3 umfasst einen zentral angeordneten Fluidkanal 20, eine damit fluchtende Düse 30, welche wiederum mit der Austrittsöffnung 31 fluchtet und radial am Umfang um die Austrittsöffnung 31 verteilte erste Ausnehmungen 33 zur Aufnahme der ersten Lichtquellen 50.

Die im Wesentlichen zylindrische Düse 30 ist in bekannter Art und Weise im vorderen Ende 3 des Handstücks 1 aufgenommen und seine Position in Strahlrichtung ist durch einen vorderen Anschlag 32 bestimmt. Die Düse 30 weist einen darin zentral angeordneten Düsenkanal 21 auf. Der Ausgestaltung dieses Kanals bestimmt die Austrittsgeometrie des Fluidstrahls 4.

Die ersten Lichtquellen 50 sind vorzugsweise UV-C Leuchtdioden mit rundem Querschnitt.

Anschliessend an die ersten Ausnehmungen 33 für die ersten Lichtquellen 50 sind fünfte Ausnehmungen 37 für die elektrische Versorgung der ersten Lichtquellen 50, sogenannte Lichtquellenleitungen 55, vorgesehen. Vorzugsweise werden die Lichtquellenleitungen 55 beim hinteren Ende 7 zusammengeführt und in einer gemeinsamen Elektroleitung 9 zu einer nicht dargestellten Versorgungseinheit geführt. Die nachfolgenden Ausführungsformen des erfindungsgemässen Handstücks 1 weisen ebenfalls fünfte Ausnehmungen 37 zur Aufnahme der Lichtquellenleitungen 55 auf.

Die ersten Lichtquellen 50 sind bezüglich der vorderen Stirnfläche 38 zu dieser zurückversetzt angeordnet. Um das Licht der ersten Lichtquellen 50 möglichst nicht zu behindern, sind die ersten Ausnehmungen 33 in ihren vorderen Bereichen konisch ausgebildet. Dadurch wird erreicht, dass der Fluidstrahl 4 baldmöglichst nach dem Austritt aus der Austrittsöffnung 31 von Licht der ersten Lichtquelle 50 umgeben ist. Auch bei kurzen Bearbeitungsdistanzen zur Wunde werden die entstehenden Aerosole somit bestrahlt.

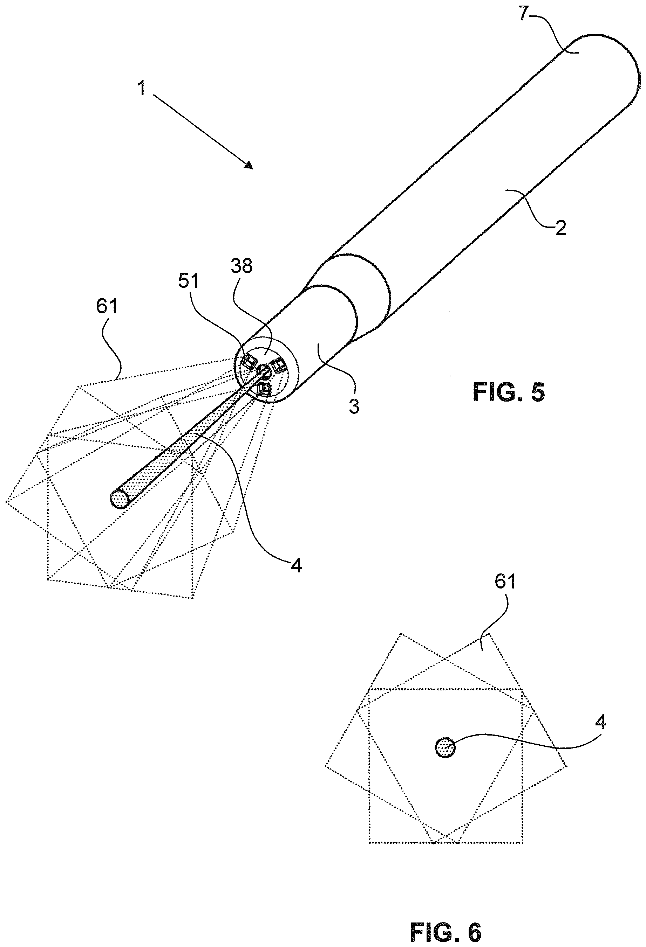

Die Figuren 5, 7 und 8 zeigen Darstellungen einer zweiten Ausführungsform eines erfindungsgemässen Systems zur Reinigung von Wunden mit Fluidstrahl, mit zweiten Lichtkegeln 61 und Fluidstrahl 4, wobei das System in dieser Ausführungsform ein Handstück 1 ist. Die Figur 6 zeigt eine Darstellung einer durch das Handstück 1 gemäss den Figuren 5, 7 und 8 beleuchteten Fläche mit dem Fluidstrahl 4.

Es handelt sich bei den zweiten Lichtquellen 51 der zweiten Ausführungsform um UV-C Leuchtdioden, welche auf einer Leiterplatte (PCB, Printed Circuit Board) angeordnet sind.

Die zweiten Lichtquellen 51 weisen einen rechteckigen Querschnitt auf und sind in entsprechenden rechteckigen zweiten Ausnehmungen 34 angeordnet. Die zweiten Lichtquellen 51 sind in diesem Beispiel zur vorderen Stirnfläche 38 zurückversetzt angeordnet.

Die Figur 6 zeigt eine Darstellung einer durch das Handstück 1 gemäss der zweiten Ausführungsform beleuchteten Fläche mit dem Fluidstrahl 4. Im Unterschied zur ersten Ausführungsform bilden die zweiten Ausnehmungen 34 zur Aufnahme der zweiten Lichtquellen 51 Lichtaustrittsbereiche, welche im Wesentlichen quadratisch ausgebildet sind. Daher weisen die Lichtkegel 61 eine pyramidenähnliche Form auf. Die durch eine zweite Lichtquelle 51 bestrahlte Fläche weist daher eine im Wesentlichen rechteckige Form auf. Die zweiten Lichtkegel 61 umhüllen den Fluidstrahl 4 bzw. er verläuft innerhalb des emittierten Lichts.

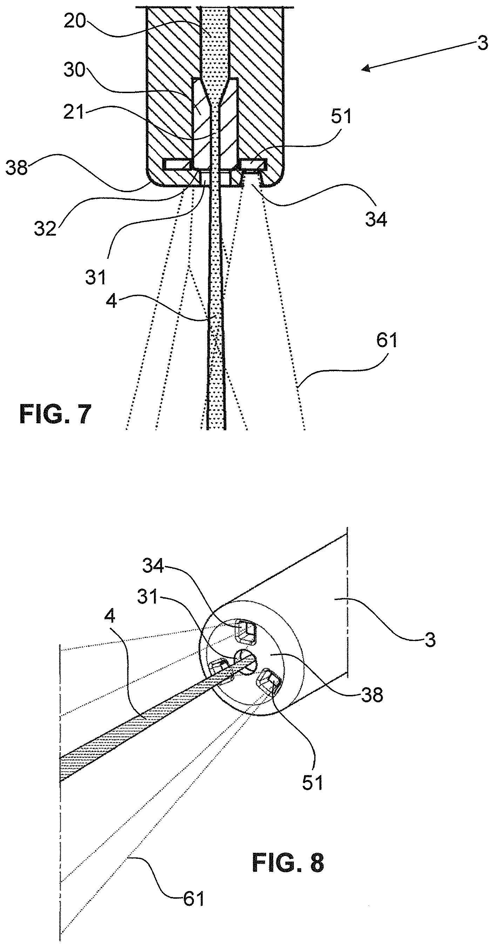

Wie in Figur 7 dargestellt, weiten sich die zweiten Ausnehmungen 34 in Strahlrichtung. Die Aussparungen zur Aufnahme der zweiten Lichtquellen 51 sind derart ausgebildet, dass die Leiterplatten in ihren Randbereichen gehalten sind.

Das Handstück 1 weist, wie das der ersten Ausführungsform, Ausnehmungen für die elektrische Versorgungsleitungen der zweiten Lichtquellen 51 auf. Ebenfalls weist das Handstück 1 in seinem hinteren Ende 7 eine Fluidleitung 8 und eine Elektroleitung 9 auf, welche hier nicht dargestellt sind.

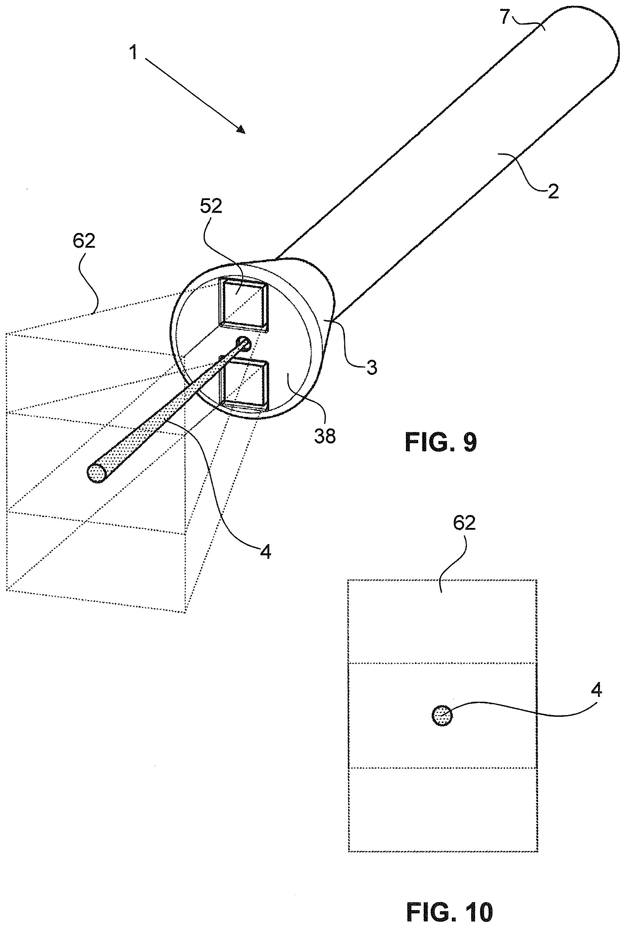

Die Figuren 9, 11 und 12 zeigen Darstellungen einer dritten Ausführungsform eines erfindungsgemässen Systems zur Reinigung von Wunden mit Fluidstrahl, mit dritten Lichtkegeln 62 und Fluidstahl 4, wobei das System in dieser Ausführungsform ein Handstück 1 ist. Die Figur 10 zeigt ein entsprechendes Beleuchtungsbild der dritten Ausführungsform.

Das Handstück 1 der dritten Ausführungsform weist ein in Strahlrichtung geweitetes vorderes Ende 3 zur Aufnahme zweier rechteckiger dritter Lichtquellen 52 auf. Die Weitung kann wie dargestellt gleichmässig am Umfang sein, sie kann jedoch in einem anderen Ausführungsbeispiel so ausgebildet sein, dass die Weitung nur im Bereich der Lichtquellen vorhanden ist. Bei den dritten Lichtquellen 52 handelt es sich um UV-C Leuchtdioden, welche in einer Leiterplatte (ICB, Integrated Circuit Board) integriert sind.

Die dritten Lichtquellen 52 weisen einen rechteckigen Querschnitt auf und sind in entsprechenden, rechteckigen dritten Ausnehmungen 35 angeordnet. Die dritten Lichtquellen 52 sind mit der vorderen Stirnfläche 38 fluchtend angeordnet.

Die Figur 10 zeigt eine Darstellung einer durch das Handstück 1 gemäss der dritten Ausführungsform beleuchteten Fläche mit dem Fluidstrahl 4. Die dritten Ausnehmungen 35 zur Aufnahme der dritten Lichtquellen 52 bilden Lichtaustrittsbereiche, welche im Wesentlichen rechteckig ausgebildet sind. Die dritten Lichtkegel 62 weisen daher eine pyramidenähnliche Form auf und die durch eine dritte Lichtquelle 52 bestrahlte Fläche weist daher eine im Wesentlichen rechteckige Form auf. Die dritten Lichtkegel 62 umhüllen den Fluidstrahl 4.

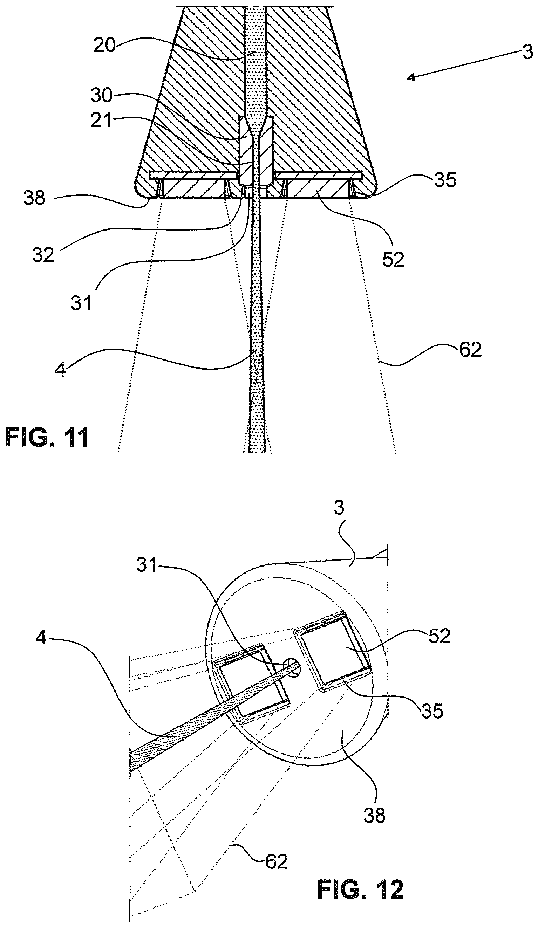

Wie in Figur 11 dargestellt, weiten sich die dritten Ausnehmungen 35 in Strahlrichtung. Die Aussparungen zur Aufnahme der dritten Lichtquellen 52 sind derart ausgebildet, dass sie die Leiterplatten in deren Randbereich zurückhalten.

Das Handstück 1 weist, wie das der ersten Ausführungsform, Ausnehmungen für die elektrische Versorgungsleitungen der dritten Lichtquellen 52 auf. Ebenfalls weist das Handstück 1 in seinem hinteren Ende 7 eine Fluidleitung 8 und eine Elektroleitung 9 auf, welche hier nicht dargestellt sind.

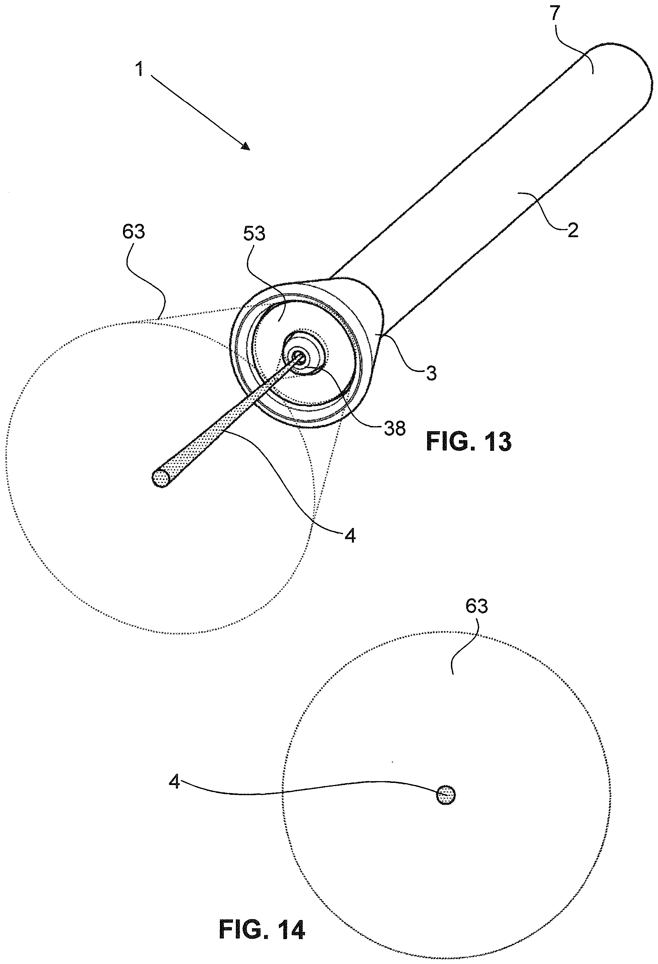

Die Figuren 13, 15 und 16 zeigen Darstellungen einer vierten Ausführungsform eines erfindungsgemässen Systems zur Reinigung von Wunden mit Fluidstrahl, mit viertem Lichtkegeln 63 und Fluidstahl 4, wobei das System in dieser Ausführungsform ein Handstück 1 ist. Die Figur 14 zeigt ein entsprechendes Beleuchtungsbild der vierten Ausführungsform.

Das Handstück der vierten Ausführungsform weist ein in Strahlrichtung geweitetes vorderes Ende 3 zur Aufnahme einer torusförmigen vierten Lichtquelle 53 auf. Die Weitung ist gleichmässig am Umfang verteilt. Bei der vierten Lichtquelle 53 handelt es sich um einen UV-C Leuchtkörper, welcher einen torusförmigen vorderen Teil und einen an dessen Rückseite anschliessenden plattenförmigen Teil aufweist. Der plattenförmige Teil weist im Wesentlichen die Form einer Kreisscheibe auf. Beispielsweise ist die vierte Lichtquelle 53 eine UV-C Quecksilberlampe oder eine LED.

Die vierten Lichtquellen 53 sind in einer entsprechenden vierten Ausnehmung 36 angeordnet. Das vordere Ende der vierten Lichtquelle 53 ist mit der vorderen Stirnfläche 38 fluchtend angeordnet.

Die Figur 14 zeigt eine Darstellung einer durch das Handstück 1 gemäss der vierten Ausführungsform beleuchteten Fläche mit dem Fluidstrahl 4. Der vierte Lichtkegel 63 weist eine kegelähnliche Form auf und die durch eine vierte Lichtquelle 53 bestrahlte Fläche weist daher eine im Wesentlichen runde Form auf. Der vierte Lichtkegel 6 umhüllt den Fluidstrahl 4.

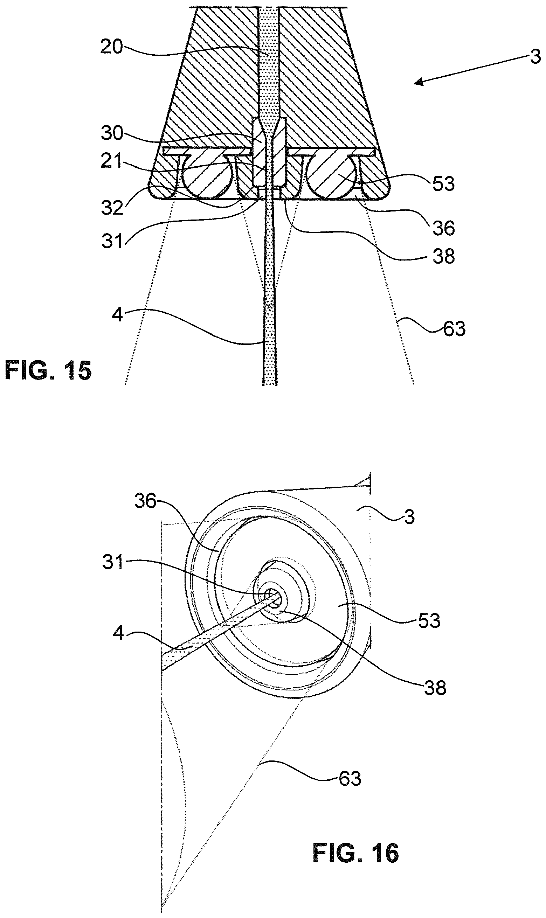

Wie in Figur 15 dargestellt, weitet sich die vierte Ausnehmung 36 in Strahlrichtung. Die Aussparung zur Aufnahme der vierten Lichtquelle 53 ist derart ausgebildet, dass sie diese in deren plattenförmigen Teil zurückhalten.

Das Handstück 1 weist, wie das der ersten Ausführungsform, Ausnehmungen für die elektrische Versorgungsleitungen der vierten Lichtquelle 53 auf. Ebenfalls weist das Handstück 1 in seinem hinteren Ende 7 eine Fluidleitung 8 und eine Elektroleitung 9 auf, welche hier nicht dargestellt sind.

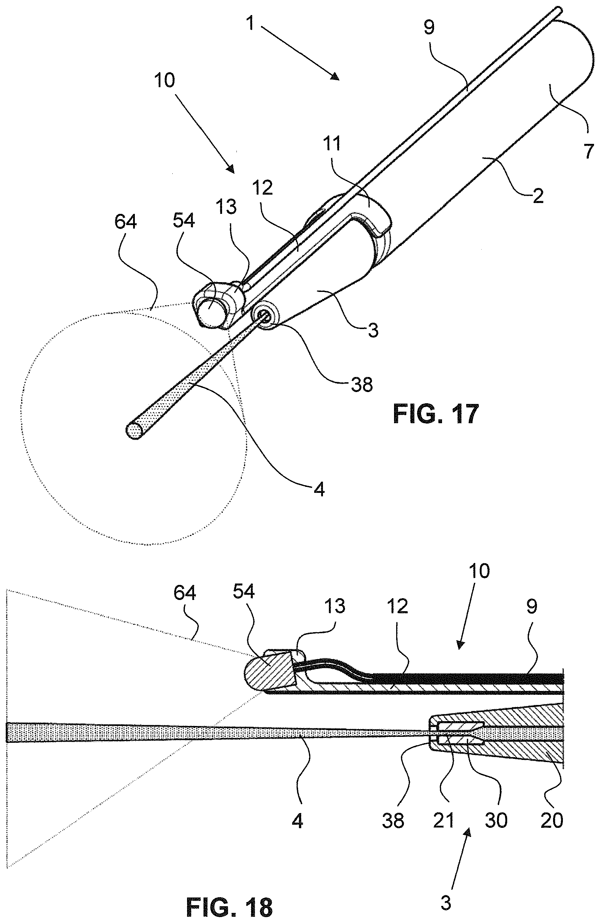

Die Figuren 17 und 18 zeigen Darstellung einer fünften Ausführungsform eines erfindungsgemässen Systems zur Reinigung von Wunden mit Fluidstrahl, mit fünftem Lichtkegel 64 und Fluidstrahl 4.

Die fünfte Ausführungsform weist ein Handstück 1 und einen an diesem Handstück 1 angeordneten Halter 10 auf. Der Halter 10 ist an einem vorderen Ende 3 des Handstücks 1 durch Klemmbacken 11 klemmend, lösbar mit diesem verbunden. Anschliessend an die Klemmbacken 11 ist ein Ausleger 12 vorgesehen, welcher sich im montierten Zustand des Halters 10 im Wesentlichen in Strahlrichtung erstreckt. Anschliessend an den Ausleger 12 ist eine Lichtquellenaufnahme 13 zur Aufnahme einer fünften Lichtquelle 54 vorgesehen.

Bei der fünften Lichtquelle 54 handelt es sich vorzugsweise um UV-C Leuchtdioden mit rundem Querschnitt. Der Durchmesser der fünften Lichtquelle 54 ist um ein Vielfaches grösser als der Durchmesser des Fluidstrahls 4. Vorzugsweise ist er mindestens fünfmal grösser, vorzugsweise mindestens zehnmal grösser.

Wie in Figur 18 dargestellt, ragt der Ausleger 12 des Halters 10 über eine vordere Stirnfläche 38 des Handstücks 1. Die fünfte Lichtquelle 54 ist somit in Strahlrichtung beabstandet zur vorderen Stirnfläche 38 angeordnet. Der fünfte Lichtkegel 64 weist eine kegelähnliche Form auf und umhüllt den Fluidstrahl 4.

Das Handstück 1 weist in seinem hinteren Ende 7 eine Fluidleitung 8 auf, welche hier nicht dargestellt sind. Die fünfte Lichtquelle 54 wird durch eine Elektroleitung 9 gespeist, welche die fünfte Lichtquelle 54 mit einer externen Stromquelle verbindet.

Das erfindungsgemässe Handstück ermöglicht eine Wundreinigung bei gleichzeitiger Dekontamination der entstehenden Aerosole, so dass die Gefahr einer Kontaminierung der Umgebung verringert ist. Vorzugsweise wird die Wunde gleichzeitig mit UV-Licht behandelt.

### BEZUGSZEICHENLISTE

| | | | |
|---|---|---|---|
| 1 | Handstück | | |
| 10 | Halter | 4 | Fluidstrahl |
| 11 | Klemmbacke | 50 | Erste Lichtquelle |
| 12 | Ausleger | 51 | Zweite Lichtquelle |
| 13 | Lichtquellenaufnahme | 52 | Dritte Lichtquelle |
| 2 | Grundkörper | 53 | Vierte Lichtquelle |
| 20 | Fluidkanal | 54 | Fünfte Lichtquelle |
| 21 | Düsenkanal | 55 | Lichtquellenleitung |
| 3 | Vorderes Ende | 60 | Erster Lichtkegel |
| 30 | Düse | 61 | Zweiter Lichtkegel |
| 31 | Austrittsöffnung | 62 | Dritter Lichtkegel |
| 32 | Vorderer Anschlag | 63 | Vierter Lichtkegel |
| 33 | Erste Ausnehmung | 64 | Fünfter Lichtkegel |
| 34 | Zweite Ausnehmung | 7 | Hinteres Ende |
| 35 | Dritte Ausnehmung | 8 | Fluidleitung |
| 36 | Vierte Ausnehmung | 9 | Elektroleitung |
| 37 | Fünfte Ausnehmung | | |
| 38 | Vordere Stirnfläche | | |

## Patentansprüche

1. System zur Behandlung von Wunden mit einem Fluidstrahl (4) mit einem Handstück (1), welches einen Grundkörper (2) mit einem vorderen Ende (3) mit einer Austrittsöffnung (31) zum Austritt des Fluidstrahls (4) aufweist, **dadurch gekennzeichnet, dass** das System ferner mindestens eine Licht aussendende Lichtquelle (50, 51, 52, 53, 54) aufweist, wobei die Lichtquelle eine UV Lichtquelle ist, welche bei der Reinigung der Wunde entstehende Aerosole bestrahlt, dass die mindestens eine Lichtquelle (50, 51, 52, 53, 54) derart angeordnet ist, dass das von der mindestens einen Lichtquelle (50, 51, 52, 53, 54) ausgesandte Licht den aus der Austrittsöffnung (31) ausgetretenen Fluidstrahl (4) mindestens in einem Abstand zur Austrittsöffnung (31) umgibt, wobei die mindestens eine Lichtquelle (50, 51, 52, 53, 54) derart bezüglich der Austrittsöffnung (31) angeordnet ist, dass der ausgetretene Fluidstrahl (4) vollständig vom ausgesandten Licht umgeben ist.

2. System nach Anspruch 1, wobei die mindestens eine Lichtquelle (50, 51, 52, 53, 54) im oder am Handstück (1) angeordnet ist.

3. System gemäss einem der Ansprüche 1 oder 2, wobei das Handstück (1) ein vorderes Ende (3) aufweist, wobei das vordere Ende (3) die Austrittsöffnung (31) aufweist.

4. System gemäss Anspruch 3, wobei die Austrittsöffnung (31) zentrisch im vorderen Ende (3) angeordnet ist.

5. System gemäss einem der Ansprüche 3 oder 4, wobei die mindestens eine Lichtquelle (50, 51, 52, 53, 54) an oder in einem fluidstrahlaustrittsnahen Bereich des vorderen Endes (3) angeordnet ist.

6. System gemäss einem der Ansprüche 3 bis 5, wobei die mindestens eine Lichtquelle (50, 51, 52, 53, 54) die Austrittsöffnung (31) umgebend am oder im vorderen Ende angeordnet ist.

7. System nach einem der Ansprüche 3 bis 6, wobei die mindestens eine Lichtquelle in einem Adapterteil auf das vordere Ende des Handstücks aufgesteckt ist.

8. System gemäss einem der Ansprüche 3 bis 7, wobei das vordere Ende (3) eine Ausnehmung (33, 34, 35, 36) zur Aufnahme der mindestens einen Lichtquelle (50, 51, 52, 53, 54) aufweist, wobei sich die Ausnehmung (33, 34, 35, 36) in Austrittsrichtung des Fluidstrahls (4) weitet.

9. System gemäss einem der Ansprüche 3 bis 8, wobei das vordere Ende (3) einen Lichtleiter aufweist, welcher von der mindestens einen Lichtquelle (50, 51, 52, 53, 54) emittiertes Licht in Richtung des Fluidstrahls (4) ausstrahlt.

10. System gemäss einem der Ansprüche 3 bis 9, wobei das vordere Ende (3) eine Düse (30) aufweist, welche die Austrittsöffnung (31) bildet und wobei das Handstück steif ausgebildet ist.

11. System gemäss einem der Ansprüche 1 bis 10, wobei die mindestens eine Lichtquelle (50, 51, 52, 53, 54) einen Lichtaustrittsbereich aufweist, welcher rund, oval, quadratisch, rechteckig, vieleckig oder torusförmig ausgebildet ist.

12. System gemäss einem der Ansprüche 1 bis 11, wobei die Lichtquelle eine UV-C Lichtquelle ist.

13. System gemäss einem der Ansprüche 1 bis 12, wobei es ein System zur Reinigung von Wunden ist.

## Claims

1. A system for treating wounds with a fluid jet (4) with a handpiece (1), which has a main body (2) with a front end (3) with an emergence opening (31) for the emergence of the fluid jet (4), **characterized in that** the system furthermore has at least one light-emitting light source (50, 51, 52, 53, 54), wherein the light source is a UV light source, which irradiates aerosols created when cleansing the wound, **in that** the at least one light source (50, 51, 52, 53, 54) is arranged in such a way that the light emitted by the at least one light source (50, 51, 52, 53, 54) surrounds the fluid jet (4) that emerged from the emergence opening (31) at least at one distance from the emergence opening (31), wherein the at least one light source (50, 51, 52, 53, 54) is arranged in such a way in relation to the emergence opening (31) that the emerged fluid jet (4) is completely surrounded by the emitted light.

2. The system as claimed in claim 1, wherein the at least one light source (50, 51, 52, 53, 54) is arranged in or at the handpiece (1).

3. The system as claimed in one of claims 1 or 2, wherein the handpiece (1) comprises a front end (3), wherein the front end (3) comprises the emergence opening.

4. The system of claim 3, wherein the emergence opening (31) is arranged centrally in the front end (3).

5. The system as claimed in one of claims 3 or 4, wherein the at least one light source (50, 51, 52, 53, 54) is arranged at or in a region of the front end (3) in the vicinity of where the fluid jet emerges.

6. The system as claimed in one of claims 3 to 5, wherein the at least one light source (50, 51, 52, 53, 54) is arranged in a manner surrounding the emergence opening (31) at or in the front end.

7. The system as claimed in one of claims 3 to 6, wherein the at least one light source is plugged onto the front end of the handpiece in an adapter part.

8. The system as claimed in one of claims 3 to 7, wherein the front end (3) has a recess (33, 34, 35, 36) for receiving the at least one light source (50, 51, 52, 53, 54), wherein the recess (33, 34, 35, 36) expands in the emergence direction of the fluid jet (4).

9. The system as claimed in one of claims 3 to 8, wherein the front end (3) has an optical waveguide which radiates light emitted by the at least one light source (50, 51, 52, 53, 54) in the direction of the fluid jet (4).

10. The system as claimed in one of claims 3 to 9, wherein the front end (3) has a nozzle (30), said nozzle forming the emergence opening (31) and wherein the handpiece has a rigid embodiment.

11. The system as claimed in one of claims 1 to 10, wherein the at least one light source (50, 51, 52, 53, 54) has a light-emergence region which has a round, oval, quadratic, rectangular, polygonal or toroidal embodiment.

12. The system as claimed in one of claims 1 to 11, wherein the light source is a UV-C light source.

13. The system as claimed in one of claims 1 to 12, wherein it is a system for cleansing wounds.

## Revendications

1. Système de traitement de blessures avec un jet de fluide (4), comprenant une pièce à main (1) qui possède un corps de base (2) doté d'une extrémité avant (3) ayant une ouverture de sortie (31) destinée à la sortie du jet de fluide (4), **caractérisé en ce que** le système possède en outre au moins une source de lumière (50, 51, 52, 53, 54) qui émet de la lumière, la source de lumière étant une source de lumière UV qui irradie les aérosols produits lors du nettoyage de la blessure, **en ce que** l'au moins une source de lumière (50, 51, 52, 53, 54) est disposée de telle sorte que la lumière émise par l'au moins une source de lumière (50, 51, 52, 53, 54) entoure le jet de fluide (4) sortant de l'ouverture de sortie (31) au moins à un écart donné de l'ouverture de sortie (31), l'au moins une source de lumière (50, 51, 52, 53, 54) étant disposée par rapport à l'ouverture de sortie (31) de telle sorte que le jet de fluide (4) sortant est entièrement entouré par la lumière émise.

2. Système selon la revendication 1, l'au moins une source de lumière (50, 51, 52, 53, 54) étant disposée dans ou sur la pièce à main (1).

3. Système selon l'une des revendications 1 ou 2, la pièce à main (1) possédant une extrémité avant (3), l'extrémité avant (3) possédant l'ouverture de sortie (31).

4. Système selon la revendication 3, l'ouverture de sortie (31) étant disposée centrée dans l'extrémité avant (3).

5. Système selon l'une des revendications 3 ou 4, l'au moins une source de lumière (50, 51, 52, 53, 54) étant disposée sur ou dans une zone de l'extrémité avant (3) proche de la sortie du jet de fluide.

6. Système selon l'une des revendications 3 à 5, l'au moins une source de lumière (50, 51, 52, 53, 54) étant disposée sur ou dans l'extrémité avant en entourant l'ouverture de sortie (31).

7. Système selon l'une des revendications 3 à 6, l'au moins une source de lumière étant emmanchée dans une pièce d'adaptation sur l'extrémité avant de la pièce à main.

8. Système selon l'une des revendications 3 à 7, l'extrémité avant (3) possédant une cavité (33, 34, 35, 36) destinée à accueillir l'au moins une source de lumière (50, 51, 52, 53, 54), la cavité (33, 34, 35, 36) s'élargissant dans le sens de la sortie du jet de fluide (4).

9. Système selon l'une des revendications 3 à 8, l'extrémité avant (3) possédant un guide de lumière qui rayonne la lumière émise par l'au moins une source de lumière (50, 51, 52, 53, 54) en direction du jet de fluide (4).

10. Système selon l'une des revendications 3 à 9, l'extrémité avant (3) possédant une buse (30) qui forme l'ouverture de sortie (31) et la pièce à main étant de configuration rigide.

11. Système selon l'une des revendications 1 à 10, l'au moins une source de lumière (50, 51, 52, 53, 54) possédant une zone de sortie de lumière qui est de configuration ronde, ovale, carrée, rectangulaire, polygonale ou toroïdale.

12. Système selon l'une des revendications 1 à 11, la source de lumière étant une source de lumière UV-C.

13. Système selon l'une des revendications 1 à 12, le système étant un système pour le nettoyage des blessures.
